# EUROPÄISCHE PATENTSCHRIFT

(11) **EP 0 297 028 B1**
(45) Veröffentlichungstag und Bekanntmachung des Hinweises auf die Patenterteilung: **18.08.1993**
(21) Anmeldenummer: 88810263.9
(22) Anmeldetag: 26.04.1988
(51) Int. Cl.: C07C 43/29, C07C 41/16

(54) **Verfahren zur Herstellung chlorierter Diphenylether**
Process for the preparation of chlorinated diphenyl ether
Procédé pour la préparation de l'éther diphényle chloré

(30) Priorität: 04.05.1987 US 46594
(43) Veröffentlichungstag der Anmeldung: 28.12.1988
(73) Patentinhaber: CIBA-GEIGY AG, 4002 Basel (CH)
(72) Erfinder: Rauber, Peter, Dr., CH-4441 Thürnen (CH)

(56) Entgegenhaltungen:
- EP-A- 0 051 235
- FR-A- 2 197 849
- US-A- 3 472 782

## Beschreibung

Die Erfindung betrifft ein neues Verfahren zur Herstellung von Chlordiphenylethern.

Chlorierte Diphenylether sind wertvolle chemische Zwischenprodukte im Pharma- und Pflanzenschutzbereich (z.B. EP-A-0051235, EP-A-0065485 und EP-A-126 430). Darüber hinaus werden Chlordiphenylether auch als Wuchsregulatoren (US-P 4 124 370) oder Hydrauliköle (US-P 3 371 120, US-P 3 472 782) vorgeschlagen.

Zur Herstellung von Diarylethern, insbesondere von Diphenylethern ist eine Reihe verschiedener Methoden bekannt. Ein vorteilhaftes Verfahren ist die sogenannte Ullmann-Reaktion (vgl. Krauch-Kunz, Reaktionen der organischen Chemie, Dr. A. Hüthig Verlag Heidelberg, 1976, S. 320). Diese besteht in der Umsetzung von Alkaliphenolaten mit Arylhalogeniden in Gegenwart von Kupfer oder Kupferverbindungen als Katalysator bei erhöhter Temperatur.

Die Ullmann-Reaktion ist ihrerseits wiederum in einer Reihe unterschiedlicher Ausführungsformen bekannt. Allgemein bedarf es bei der Ullmann-Reaktion eines aktivierten Halogenaromaten, wie etwa Jod-oder Brombenzole. Nach Houben-Weyl (Methoden der organischen Chemie, G. Thieme Verlag Stuttgart 1965, Band VI.3., S. 86) reagieren Bromverbindungen (Arylbromide) leichter als die entsprechenden Chlorverbindungen.

So ist die Herstellung von 2-, 3- und 4-Chlordiphenylether aus 2-bzw. 3-Chlor-iod-benzol oder Brombenzol bekannt geworden (J. Amer. Chem. Soc.; 59 [1937] 2578 und Gazz. Chim. Ital.; 86 [1956] 1248). Brombenzol oder Iodbenzol, sowie die gemischten Chlor-brom- oder Chlor-iod-benzole sind weniger gut verfügbar als Chlorbenzol oder die isomeren Dichlorbenzole.

Es sind daher verschiedene Verfahrenvarianten der Ullmann-Reaktion vorgeschlagen worden, welche auf der Umsetzung der an sich weniger reaktiven Dichlorbenzolen mit Phenolat oder Chlorphenolat beruhen. In den US-Patentschriften US-P 3 472 782 und US-P 3 371 120 wird 1,4-Dichlorbenzol mit 3-Chlorphenolat bei 165°C unter CuCl/KJ-Katalyse zu 3,4'-Dichlordiphenylether umgesetzt. In der FR-A-2 197 849 wird die Umsetzung von Phenolaten mit Dichlorbenzolen und einem Kupferkatalysator in Gegenwart des freien Phenols vorgeschlagen.

Eine Verbesserung des Ullmann-Katalysators wird in der EP-A-0051235 mit der Verwendung basischer Kupfercarbonate oder Kupfersalze niederer aliphatischer Carbonsäuren und Phenolüberschuss vorgeschlagen. Dieses, sowie die vorgenannten Verfahren befriedigen nicht im Hinblick auf Ausbeute, Reaktionsführung und Reaktionszeit. So wird aufgrund eigener Untersuchungen gefunden, dass 4-Chlorphenolat mit 1,3-Dichlorbenzol selbst bei 24 stündiger Reaktionszeit bei 150°C am Kupferkontakt (CuO) nur in 10-20 % Ausbeute zu 3,4'-Dichlor-diphenylether reagiert.

Des weiteren ist bekannt, dass bei der Verwendung von Dimethylformamid als Lösungsmittel die Ullmann-Reaktion bei aktivierten Halogenbenzolen in vorteilhafter Weise durchgeführt werden kann. Es ist unter anderem die Umsetzung von Nitrophenolaten mit Chlornitrobenzolen zu den entsprechenden Dinitrodiphenylethern beschrieben worden (J. Org. Chem.; 27 [1962] 4098). An anderer Stelle (J. Amer. Chem. Soc. 74 [1952] 5782) wird darauf hingewiesen, dass die Verwendung wenig reaktiver Halogenbenzole als Edukte und Dimethylformamid als Lösungsmittel keine Verbesserung der bekannt geringen Produktausbeuten ergibt.

Dies steht im Einklang mit eigenen Untersuchungen, bei denen anhand der Umsetzung von 1,3-Dichlorbenzol mit 4-Chlorphenolat am Kupferkontakt gefunden wurde, dass neben den niedrigen Umsätzen zu 3,4'-Dichlordiphenylether dieser Ether zudem mit noch im Reaktionsgemisch vorhanden 4-Chlorphenolat unter Chlor-Phenolat-Austausch zu oligomeren (vorzugsweise tri- und tetrameren) Ethern weiterreagiert.

Der Erfindung liegt somit die Aufgabe zugrunde ein Verfahren zur Synthese chlorierter Diphenylether vorzuschlagen, in welchem die an sich wenig reaktiven Dichlorbenzole mit Phenolat bzw. Chlorphenolat in guten Ausbeuten und mit guter Selektivität zu den gewünschten Produkten umgesetzt werden können.

Erfindungsgemäss wird diese Aufgabe gelöst durch ein Verfahren zur Herstellung von Chlordiphenylethern der Formel I 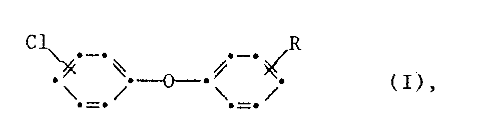
worin R für Wasserstoff oder Chlor steht, durch Umsetzung eines Phenolats der Formel III 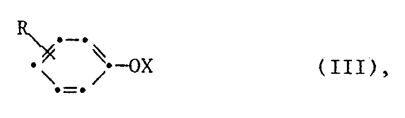
worin X für ein Aequivalent eines Alkali- oder Erdalkaliions steht und R wie zuvor definiert ist, in einem Ueberschuss eines Dichlorbenzols der Formel II 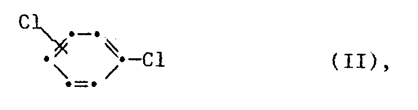
in Gegenwart eines Kupferkatalysators bei Temperaturen von 120-220°C, dadurch gekennzeichnet, dass man zusätzlich ein aprotisches Lösungsmittel als Co-Katalysator verwendet.

Aprotische Lösungsmittel sind unter anderem:
Sulfolan, Butyronitril, Amine, wie Di-n-butylamin, Aniline wie Anilin, Methylanilin und Dimethylanilin, Acetonitril, Propionitril, Pyridine, Dimethylformamid, Dimethylsulfoxid, Diethylenglycoldimethylether, 1,2-Dimethoxyethan, Hexamethylenphosphorsäuretriamid, 1-Methylpyrrolidon-2, Dimethylacetamid, Benzonitril, Formamid, Ethylencarbonat, Propylencarbonat, N-Methylformamid usw..

Bevorzugt sind Dimethylformamid, Dimethylacetamid, Dimethylsulfoxid und 1-Methylpyrrolidon-2.

Die Umsetzung wird vorzugsweise bei Normaldruck durchgeführt. Im Falle niedrig siedender Co-Katalysatoren oder wenn erhöhte Reaktionstemperaturen erwünscht sind kann die Reaktion auch bei erhöhtem Druck insbesondere bei Drücken bis zu 5 bar durchgeführt werden. Der Druck ist jedoch nicht kritisch, es kann auch bei vermindertem Druck, insbesondere bis 0,5 bar, gearbeitet werden.

Bevorzugt sind Temperaturen von 130 bis 190°C, insbesondere 140 bis 170°C. In vorteilhafter Weise kann unter Normaldruck etwas unterhalb des Siedepunkts des Reaktionsgemischs gearbeitet werden.
Die Siedetemperatur wird in erster Linie durch das im Ueberschuss vorhandene Dichlorbenzol der Formel II und dem Druck bestimmt. Besonders bevorzugt ist somit der Temperaturbereich von 150 bis 170°C.

Der als Co-Katalysator verwendete Zusatz des aprotischen Lösungsmittels kann 0,3 bis 300m Mol% (bezogen auf das Phenolat) insbesondere 5-100 Mol%, bevorzugt 20-40 Mol% betragen und wird in bestimmtem Umfang durch die anderen Verfahrensparameter (Temperatur, Co-Katalysator, Struktur des Katalysators, Verhältnis der Edukte III und II) beeinflusst. Die jeweils optimale Menge an Co-Katalysator kann durch einfache Experimente bestimmt werden. Sie beträgt bei der Verwendung von Dimethylformamid oder Dimethylacetamid (bei 150°C Reaktionstemperatur CuO als Katalysator und einem Molverhältnis von II:III = 9:1) etwa 30 Mol%.

Als Ullmann-Katalysatoren sind unter anderem zu nennen:
metallisches Kupfer (Kupferbronze, gegebenenfalls aktiviert durch Behandlung mit einer acetonischen Jodlösung und Salzsäure), CuI, CuBr, CuBr₂, CuCN, Cu(NO₃)₂, CuSO₄, CuCl, CuCl₂, Cu(OH)₂, CuCO₃-Cu(OH)₂, Cu(OCOCH₃)₂, Cu₂O oder CuO sowie die aus der EP 51 235 bekannten Cu-Carbonate und Salze niederer aliphatischer Carbonsäuren.

In besonders vorteilhafter Weise ist CuO als Katalysator geeignet.

Der Katalysator kann nach Beendigung der Reaktion zurückgewonnen und wiederverwendet werden.

Die jeweils vorteilhaft verwendbare Menge an Ullmann-Katalysator beträgt 0,5-100 Mol% (bezogen auf das Phenolat III), bevorzugt 1 bis 50 Mol%, insbesondere 1 bis 5 Mol%. Die anderen Verfahrensparameter, wie Temperatur, Co-Katalysator, Verhältnis der Edukte haben einen Einfluss auf die jeweils optimale Menge an Ullmann-Katalysator. Unter Berücksichtigung der angestrebten Raum-Zeit-Ausbeute kann die jeweils optimale Menge durch Versuche ermittelt werden. Sie beträgt bevorzugt 1 bis 2 Mol% bei der Verwendung von CuO (bei 150°C, Dimethylformamid oder Dimethylacetamid als Co-Katalysator und einem Molverhältnis II:III = 9:1).

Bei dem erfindungsgemässen Verfahren wird das Dichlorbenzol der Formel II im Ueberschuss als Lösungsmittel verwendet. In vorteilhafter Weise beträgt das Verhältnis II:III = 3:1 bis 15:1 insbesondere 5:1 bis 12:1.

Während in den aus dem Stand der Technik bekannten Verfahren das Phenolat III zunächst in einem getrennten Verfahrensschritt in einem anderen Lösungsmittel hergestellt wird, kann erfindungsgemäss die Phenolatbildung in dem als Lösungsmittel verwendeten Dichlorbenzol II direkt mit NaOH oder KOH und dem Phenol der Formel IV vorgenommen werden.

Die Salzbildung gemäss nachstehender Formel 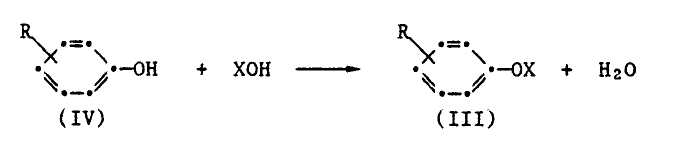
wird vorteilhaft bei Raumtemperatur oder leicht erhöhter Temperatur (bis 120°C) durchgeführt. Anschliessend wird auf Siedehitze erwärmt und das bei der Salzbildung freigesetzte Reaktionswasser mit einem Wasserabscheider ausgekreist. Dieser Verfahrensschritt wird vorzugsweise bei vermindertem Druck durchgeführt. Im Falle fester Dichlorbenzole der Formel (II) kann der Zusatz eines zeotropen Schleppmittels, wie etwa Toluol und Xylol, vorteilhaft sein, damit beim Auskreisen die Verbindung der Formel (II) nicht auskristallisiert. Die Verwendung eines Schleppmittels ist jedoch nicht unbedingt erforderlich, es kann auch durch geringfügiges Erwärmen des Wasserabscheiders der Kristallisation vorgebeugt werden. Das so erhältliche Salz der Formel III reagiert nach Zugabe des Ullmann-Katalysators und des Co-Katalysators (der auch schon bei der Salzbildung dem Reaktionsgemisch zugefügt werden kann) bei der entsprechenden Reaktionstemperatur mit dem in Ueberschuss vorhandenen Dichlorbenzol (II) in zuvor beschriebener Weise zum Produkte der Formel I.

Das als Base benötigte Natrium- bzw. Kaliumhydroxid kann in fester Form, vorteilhaft jedoch als Monohydrat oder in konzentrierter wässriger Lösung zugesetzt werden. Das überschüssige Wasser wird zusammen mit dem bei der Reaktion freigesetzten Wasser durch Auskreisen aus dem Reaktionsgemisch entfernt. Im Vergleich zu den aus dem Stand der Technik bekannten Verfahren ist die Salzbildung in dem für die Ullmann-Reaktion als Lösungsmittel verwendeten Dichlorbenzol II vorteilhaft, weil erfindungsgemäss in beiden Stufen ohne Wechsel des Lösungsmittels gearbeitet werden kann.

Mit dem erfindungsgemässen Verfahren können Chlordiphenylether der Formel I in guter Ausbeute und mit guter Selektivität bei relativ niedriger Reaktionstemperatur und Reaktionszeit hergestellt werden. Unter den zuvor genannten Reaktionsbedingungen ist die Reaktion zu I in 4 bis 15 Stunden nahezu vollständig.

In besonders vorteilhafter Weise ist die erfindungsgemässe Reaktion für die Herstellung von 3,4′-Dichlordiphenylether aus 1,3-Dichlorbenzol und 4-Chlorphenol oder 1,4-Dichlorbenzol und 3-Chlorphenol geeignet. Es ist bekannt, dass z.B. durch die meta-Substitution im 1,3-Dichlorbenzol der elektronenziehende Effekt der beiden Chlorsubstituenten aufeinander nur ausserordentlich schwach und demzufolge 1,3-Dichlorbenzol als ein für Ullmann-Reaktionen nicht aktiviertes Halogenbenzol anzusehen ist. Aehnliche Ueberlegungen gelten für 1,2-Dichlorbenzol und in besonderem Masse auch für 1,4-Dichlorbenzol. Obwohl der elektronegative Effekt des in ortho-bzw. para-Position gebundenem zweiten Chloratoms einen sehr schwach aktivierenden Einfluss auf das reaktive Ullmann-Zentrum ausüben, wird doch dieser Effekt durch einen mesomeriebedingten elektronenabgebenden Effekt so stark abgeschwächt, dass auch 1,2-und 1,4-Dichlorbenzol als nicht aktivierte Edukte im Sinne einer Ullmann-Reaktion anzusehen sind. Demzufolge ist es ausserordentlich überraschend, dass durch die Verwendung des einen Ullman-Edukts (Dichlorbenzol II) als Lösungsmittel in Kombination mit einem Co-Katalysator die Diphenylether I in guter Ausbeute und Selektivität bei kurzer Reaktionszeit und niedriger Reaktionstemperatur herstellbar sind.

Die nachstehenden Beispiele erläutern die Erfindung.

### Beispiel 1: 3,4′-Dichlordiphenylether

Zu einer Lösung von 128,1 g (1,0 Mol) 4-Chlorphenol in 1325 g (9 Mol) 1,3-Dichlorbenzol und 27 g (0,3 Mol) Dimethylacetamid werden 80 g 50%-iges Natriumhydroxid gegeben.
Unter Rühren und schwachem Vakuum (600 mbar) wird erwärmt und bei einer Innentemperatur von ca. 110°C beginnend das Wasser ausgekreist. Dabei wird die Innentemperatur kontinuierlich bis auf 150°C erhöht. Dann wird das Reaktionsgemisch mit 1,2 g CuO versetzt und unter kräftigem Rühren bei einer Innentemperatur von 150°C unter Normaldruck ausreagieren gelassen.
Nach dem Erkalten werden 200 ml Wasser zugegeben, mit NaOH auf pH 12 gestellt, über Hyflo filtriert, von der wässrigen Phase abgetrennt und die organische Phase im Hochvakuum destillativ aufgearbeitet.

Man isoliert 190 g (80 %) der Titelverbindung der Formel

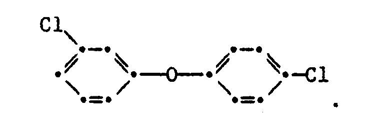

### Beispiel 2: 3,4′-Dichlordiphenylether

Zu einer Lösung von 128,1 g (1 Mol) 3-Chlorphenol in 1325 g (9 Mol) 1,4-Dichlorbenzol und 27 g (0,3 Mol) Dimethylacetamid werden 80 g (1 Mol) 50%-iges Natriumhydroxid gegeben.

Unter Rühren und schwachem Vakuum wird erwärmt und das Wasser ausgekreist (zur Vereinfachung kann als Wasserschlepper Toluol zugesetzt werden). Dann wird das Reaktionsgemisch mit 1,2 g CuO versetzt und unter kräftigem Rühren bei einer Innentemperatur von 150°C unter Normaldruck ausreagieren gelassen.
Nach wässriger Aufarbeitung und Hochvakuumdestillation der organischen Phase (analog Beispiel 1) isoliert man 180 g (75%) der Titelverbindung.

### Beispiel 3: 2,4′-Dichlordiphenylether

Zu einer Lösung von 128,1 g (1 Mol) 4-Chlorphenol in 1470 g (10 Mol) 1,2-Dichlorbenzol und 40 g (0,45 Mol) Dimethylacetamid werden 80 g (1 Mol) 50 %-iges Natriumhydroxid gegeben.
Unter Rühren und schwachem Vakuum (analog Beispiel 1) wird Wasser ausgekreist.
Dann wird das Reaktionsgemisch mit 2,3 g CuO versetzt und unter Rühren bei 150°C und Normaldruck zur Reaktion gebracht. Nack wässriger Aufarbeitung und Hochvakuumdestillation (analog Beispiel 1) isoliert man 190 g (80 %) der Titelverbindung der Formel

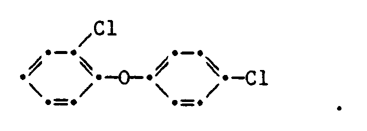

## Patentansprüche

1. Verfahren zur Herstellung von Chlordiphenylethern der Formel I 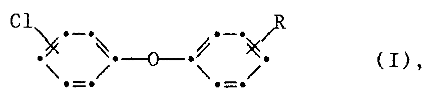 worin R für Wasserstoff oder Chlor steht, durch Umsetzung eines Phenolats der Formel III 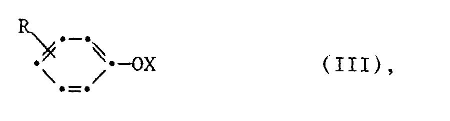 worin X für ein Aequivalent eines Alkali- oder Erdalkaliions steht und R wie zuvor definiert ist, in einem Ueberschuss eines Dichlorbenzols der Formel II 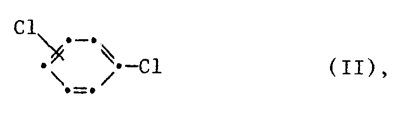 in Gegenwart eines Kupferkatalysators bei Temperaturen von 120-220°C, dadurch gekennzeichnet, dass man zusätzlich ein aprotisches Lösungsmittel als Co-Katalysator verwendet.

2. Verfahren gemäss Anspruch 1 zur Herstellung von 3,4'-Dichlordiphenylether I aus 1,3-Dichlorbenzol (II) und Natrium 4-Chlorphenolat (III).

3. Verfahren gemäss einem der Ansprüche 1 oder 2, dadurch gekennzeichnet, dass man das Phenolat der Formel III durch Umsetzung eines Chlorphenols der Formel IV 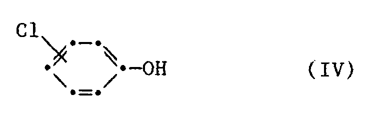 mit XOH, wobei X für ein Aequivalent eines Alkali- oder Erdalkaliions steht, in Dichlorbenzol (II) gewünschtenfalls unter Zusatz des als Co-Katalysator verwendeten aprotischen Lösungsmittels herstellt und das im Reaktionsgemisch vorhandene Wasser bei erhöhter Temperatur gewünschtenfalls unter vermindertem Druck auskreist.

4. Verfahren gemäss einem der Ansprüche 1 bis 3, dadurch gekennzeichnet, dass man die Ullmann-Reaktion bei Temperaturen von 130-190°C durchführt.

5. Verfahren gemäss Ansprüchen 1 bis 4, dadurch gekennzeichnet, dass man Sulfolan, Butyronitril, Di-n-butylamin, Anilin, Methylanilin, Dimethylanilin, Acetonitril, Propionitril, Pyridin, Dimethylformamid, Dimethylsulfoxid, Diethylenglycoldimethylether, 1,2-Dimethoxyethan, Hexamethylphosphorsäuretriamid, 1-Methylpyrrolidon-2, Dimethylacetamid, Benzonitril, Formamid, Ethylencarbonat, Propylencarbonat oder N-Methylformamid als Co-Katalysator verwendet.

6. Verfahren gemäss Anspruch 5, gekennzeichnet durch einen Gehalt von 0,3 bis 300 Mol% Co-Katalysator.

7. Verfahren gemäss einem der Ansprüche 1 bis 6, dadurch gekennzeichnet, dass man metallisches Kupfer (Kupferbronze, gegebenenfalls aktiviert durch Behandlung mit einer acetonischen Jodlösung und Salzsäure), CuI, CuCl, CuCl₂, CuBr, CuBr₂, CuCN, Cu(NO₃)₂, CuSO₄, Cu(OH)₂, CuCO₃-Cu(OH)₂, Cu(OCOCH₃)₂, Cu₂O oder CuO als Kupfer-Katalysator verwendet.

8. Verfahren gemäss Anspruch 7, gekennzeichnet durch einen Katalysator-Gehalt von 0,5-100 Mol%.

9. Verfahren gemäss Anspruch 8, gekennzeichnet durch einen Katalysator-Gehalt von 1-5 Mol%.

10. Verfahren gemäss einem der Ansprüche 1 bis 9, dadurch gekennzeichnet, dass das Verhältnis der Edukte II:III von 3:1 bis 15:1 beträgt.

## Claims

1. A process for preparing chlorodiphenyl ethers of the formula I 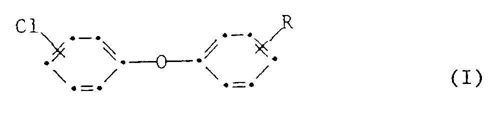 in which R is hydrogen or chlorine, by reacting a phenolate of the formula III 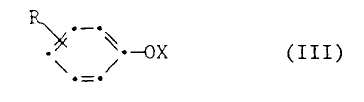 in which X is one equivalent of an alkali metal or alkaline earth metal ion and R is as defined above, in an excess of a dichlorobenzene of the formula II 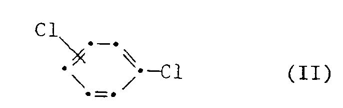 in the presence of a copper catalyst at temperatures of 120-220°C, which comprises additionally using an aprotic solvent as cocatalyst.

2. A process according to claim 1 for preparing 3,4'-dichlorodiphenyl ether I from 1,3-dichlorobenzene (II) and sodium 4-chlorophenolate (III).

3. A process according to either of claims 1 or 2, wherein the phenolate of the formula III is prepared by reacting a chlorophenol of the formula IV 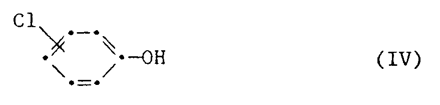 with XOH, where X is one equivalent of an alkali metal or alkaline earth metal ion, in dichlorobenzene (II) in the absence or presence of an aprotic solvent as cocatalyst, and the water present in the reaction mixture is separated out at elevated temperatures, if desired under reduced pressure.

4. A process according to any one of claims 1 to 3, wherein the Ullmann reaction is carried out at temperatures of 130-190°C.

5. A process according to claims 1 to 4, wherein the cocatalyst used is sulfolane, butyronitrile, di-n-butylamine, aniline, methylaniline, dimethylaniline, acetonitrile, propionitrile, pyridine, dimethylformamide, dimethyl sulfoxide, diethylene glycol dimethyl ether, 1,2-dimethoxyethane, hexamethylphosphoric triamide, 1-methylpyrrolid-2-one, dimethylacetamide, benzonitrile, formamide, ethylene carbonate, propylene carbonate or N-methylformamide.

6. A process according to claim 5, wherein the cocatalyst content is 0.3 to 300 mol %.

7. A process according to any one of claims 1 to 6, wherein the copper catalyst used is metallic copper (copper bronze, if desired activated by treatment with an acetonic iodine solution and hydrochloric acid), CuI, CuCl, CuCl₂_{,} CuBr, CuBr₂_{,} CuCN, Cu(NO₃)₂, CuSO₄, Cu(OH)₂, CuCO₃-Cu(OH)₂, Cu(OCOCH₃)₂_{,} Cu₂O or CuO.

8. A process according to claim 7, wherein the catalyst content is 0.5-100 mol %.

9. A process according to claim 8, wherein the catalyst content is 1-5 mol %.

10. A process according to any one of claims 1 to 9, wherein the ratio of starting compounds II:III ranges from 3:1 to 15:1.

## Revendications

1. Procédé pour la préparation des éthers chlorodiphényliques de formule I : 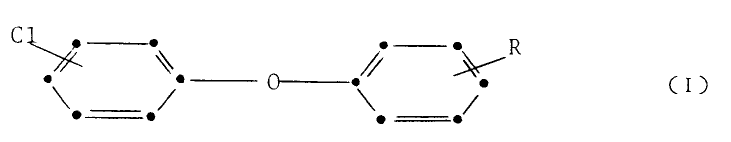 où R représente l'hydrogène ou le chlore, par réaction d'un phénolate de formule III : 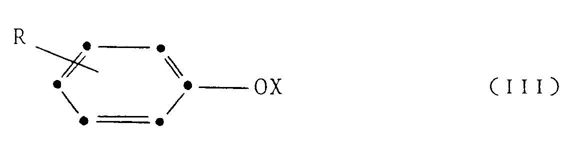 où X représente un équivalent d'un ion alcalin ou alcalino-terreux et R est défini comme précédemment, dans un excès d'un dichlorobenzène de formule II : 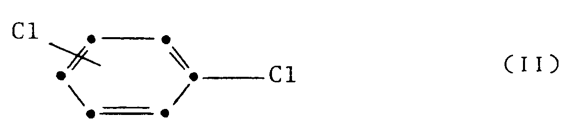 en présence d'un catalyseur à base de cuivre à des températures allant de 120 à 220°C, caractérisé en ce que l'on utilise en plus un solvant aprotique.

2. Procédé selon la revendication 1, pour la préparation de l'éther 3,4'-dichlorodiphénylique I à partir du 1,3-dichlorobenzène II et du 4-chlorophénolate de sodium III.

3. Procédé selon l'une des revendications 1 et 2, caractérisé en ce que l'on prépare le phénolate de formule III par réaction d'un chlorophénol de formule IV : 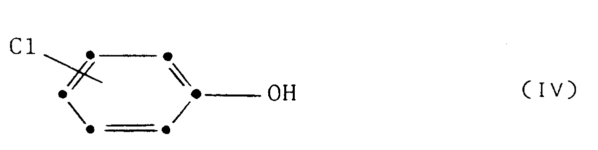 avec XOH, X représentant un équivalent d'un ion alcalin ou alcalino-terreux, dans le dichlorobenzène (II), si dèsiré, en additionnant le solvant aprotique utilisé comme co-catalyseur et en éliminant du cycle l'eau présente dans le mélange réactionnel à une température augmentée, si désiré, sous pression réduite.

4. Procédé selon l'une des revendications 1 à 3, caractérisé en ce que l'on effectue la réaction d'Ullmann à des températures allant de 130-190°C.

5. Procédé selon les revendications 1 à 4, caractérisé en ce que l'on utilise comme co-catalyseur le Sulfolane, le butyronitrile, la di-n-butylamine, l'aniline, la méthylaniline, la diméthylaniline, l'acétonitrile, le propionitrile, la pyridine, le diméthylformamide, le diméthylsulfoxyde, le diéthylèneglycoldiméthyléther, le 1,2-diméthoxyéthane, l'hexaméthylphosphorotriamide, la 1-méthylpyrrolidone-2, le diméthylacétamide, le benzonitrile, le formamide, l'éthylènecarbonate, le propylènecarbonate ou le N-méthylformamide.

6. Procédé selon la revendication 5, caractérisé en ce que la teneur en co-catalyseur est comprise entre 0,3 et 300 moles %.

7. Procédé selon l'une des revendications 1 à 6, caractérisé en ce que l'on utilise du cuivre métallique (bronze de cuivre éventuellement activé par traitement avec une solution acétonique d'iode et d'acide chlorhydrique), CuI, CuCl, CuCl₂, CuBr, CuBr₂, CuCN, Cu(NO₃)₂, CuSO₄, Cu(OH)₂, CuCO₃-Cu(OH)₂, Cu(OCOCH₃)₂, Cu₂O ou CuO, comme catalyseur à base de cuivre.

8. Procédé selon la revendication 7, caractérisé en ce que la teneur en catalyseur est comprise entre 0,5 et 100 moles %.

9. Procédé selon la revendication 8, caractérisé en ce que la teneur en catalyseur est comprise entre 1 et 5 moles %.

10. Procédé selon l'une des revendications 1 à 9, caractérisé en ce que le rapport des éductes II:III est compris entre 3:1 et 15:1.
